# EUROPEAN PATENT APPLICATION

(11) **EP 1 731 494 A1**
(43) Date of publication of application: **13.12.2006**
(21) Application number: 05727497.9
(22) Date of filing: 25.03.2005
(51) Int. Cl.: C07C 45/59, C07C 47/277

(54) **METHOD FOR PRODUCING HALOGENATED UNSATURATED CARBONYL COMPOUND**

(30) Priority: 31.03.2004 JP 2004104866
(71) Applicant: Kuraray Co., Ltd., Kurashiki Plant, Okayama 710-8622 (JP); DAIICHI PHARMACEUTICAL CO., LTD., Chuo-ku, Tokyo 103-8234 (JP)
(72) Inventor: KOYAKUMARU, Kenichi, Kuraray Chemical Co., Ltd., Bizen-shi, Okayama 7050025 (JP); HAYASHIBARA, Tatsuhiko, KURARAY CO., LTD., Kurashiki-shi, Okayama 7100801 (JP); AKIBA, Toshifumi, DAIICHI PHARMA. CO., LTD., Tokyo 1348630 (JP); SAITO, Tatsuru, DAIICHI PHARMACEUTICAL CO., LTD., Tokyo 1348630 (JP)
(74) Representative: Duckworth, Timothy John
(86) International application number: PCT/JP2005/006414
(87) International publication number: WO 2005/095317

(57) **Abstract**

The present invention provides a method for industrially advantageously producing a halogenated unsaturated carbonyl compound without environment problems. A production method of a halogenated unsaturated carbonyl compound represented by the formula (III) is provided, which includes reacting an alkoxy-cyclic ether represented by the formula (I) with an acid halide represented by the formula (II) 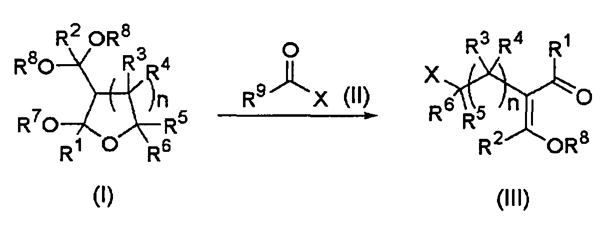 wherein each symbol is as defined in the specification.

## Description

### Technical Field

The present invention relates to methods of producing halogenated unsaturated carbonyl compounds useful as raw material for pharmaceutical products, particularly antibacterial agents.

### Background Art

As raw material for pharmaceutical products, particularly antibacterial agents, halogenated unsaturated carbonyl compounds represented by the formula (III) 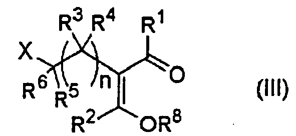 wherein R¹, R², R³, R⁴, R⁵ and R⁶ each independently represents a hydrogen atom, a saturated hydrocarbon group optionally having substituent(s), an aryl group optionally having substituent(s), an alkenyl group or an aralkyl group, R⁸ represents a saturated hydrocarbon group optionally having substituent(s), an aryl group optionally having substituent(s) or an aralkyl group, n represents 1 or 2, and X represents a halogen atom, (hereinafter sometimes to be abbreviated as halogenated unsaturated carbonyl compound (III)) are useful. Particularly, halogenated unsaturated carbonyl compound (III) wherein n is 1 is a useful compound that can be converted to cyclopropane monoacetal useful as an intermediate for synthetic antibacterial agents.

The present inventors have already found that halogenated unsaturated carbonyl compound (III) can be obtained by reacting an alkoxy-cyclic ether represented by the formula (I) 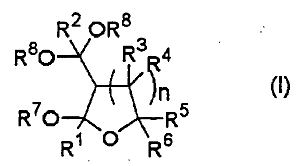 wherein R¹, R², R³, R⁴, R⁵, R⁶, R⁸ and n represent as defined above, and R⁷ represents a saturated hydrocarbon group optionally having substituent(s), an aryl group optionally having substituent(s) or an aralkyl group, (hereinafter sometimes to be abbreviated as alkoxy-cyclic ether (I)) with thionyl halide or sulfuryl halide.

While the above-mentioned reaction affords halogenated unsaturated carbonyl compound (III) economically in a good yield and at a low cost, it is associated with an environmental problem of forming by-products such as sulfurous acid gas, sulfite diester or sulfate diester.

### Disclosure of the Invention

Hence, an object of the present invention is to provide a method of industrially advantageously producing halogenated unsaturated carbonyl compound (III) without an environmental problem.

The present inventors have conducted intensive studies and found that the object halogenated unsaturated carbonyl compound (III) can be produced without forming by-products such as sulfurous acid gas, sulfite diester or sulfate diester, by reacting alkoxy-cyclic ether (I) with an acid halide represented by the below-mentioned formula (II) (hereinafter sometimes to be abbreviated as acid halide (II)), which resulted in the completion of the present invention.

Accordingly, the present invention relates to the following production methods.
[1] A method of producing a halogenated unsaturated carbonyl compound represented by the formula (III) 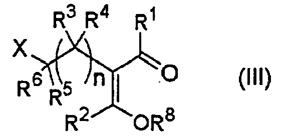 wherein R¹, R², R³, R⁴, R⁵ and R⁶ each independently represents a hydrogen atom, a saturated hydrocarbon group optionally having substituent(s), an aryl group optionally having substituent(s), an alkenyl group or an aralkyl group, R⁸ represents a saturated hydrocarbon group optionally having substituent(s), an aryl group optionally having substituent(s) or an aralkyl group, X is a halogen atom, and n is 1 or 2, which comprises reacting an alkoxy-cyclic ether represented by the formula (I) 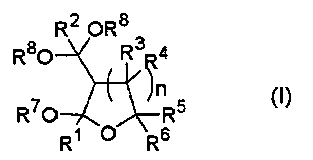 wherein R¹, R², R³, R⁴, R⁵, R⁶, R⁸ and n represent as defined above, and R⁷ represents a saturated hydrocarbon group optionally having substituent(s), an aryl group optionally having substituent(s) or an aralkyl group, with an acid halide represented by the formula (II) 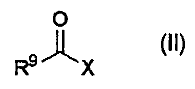 wherein R⁹ represents a saturated hydrocarbon group, an aryl group, an aralkyl group optionally having substituent(s) or a hydrocarbonoxy group, and X represents as defined above.

According to the present invention, halogenated unsaturated carbonyl compound (III) can be produced industrially advantageously without an environmental problem.

### Best Mode for Embodying the Invention

The saturated hydrocarbon group represented by R¹, R², R³, R⁴, R⁵, R⁶, R⁷ or R⁸ is linear, branched or cyclic, and the carbon number thereof is preferably 1 to 12, more preferably 1 to 6, and, for example, an alkyl group such as methyl group, ethyl group, propyl group, isopropyl group, butyl group, isobutyl group, tert-butyl group, hexyl group, octyl group, dodecyl group and the like; a cycloalkyl group such as cyclopentyl group, cyclohexyl group and the like; and the like can be mentioned. These saturated hydrocarbon groups may have substituent(s), and as such substituent, for example, an aryl group having 6 to 10 carbon atoms such as phenyl group optionally substituted by substituent(s) selected from an alkyl group having 1 to 6 carbon atoms such as methyl group and the like, an alkoxyl group having 1 to 6 carbon atoms such as methoxy group and a halogen atom such as chlorine atom and the like; an alkoxyl group having 1 to 6 carbon atoms such as methoxy group, ethoxy group, propoxy group, butoxy group and the like; and the like can be mentioned.

The aryl group represented by R¹, R², R³, R⁴, R⁵, R⁶, R⁷ or R⁸ preferably has 6 to 14, more preferably 6 to 10, carbon atoms and, for example, phenyl group, naphthyl group, anthracenyl group and the like can be mentioned. These aryl groups may have substituent(s) and as such substituent, for example, a linear, branched or cyclic saturated hydrocarbon group having 1 to 12 carbon atoms, such as methyl group, ethyl group, propyl group, isopropyl group, butyl group, isobutyl group, tert-butyl group, hexyl group, octyl group, dodecyl group, cyclopentyl group, cyclohexyl group and the like; an aryl group having 6 to 18 carbon atoms optionally having substituent(s) (e.g., an alkyl group having 1 to 3 carbon atoms, an alkoxyl group having 1 to 3 carbon atoms, a halogen atom, nitro group and the like), such as phenyl group, tolyl group, methoxyphenyl group, chlorophenyl group, bromophenyl group, nitrophenyl group, naphthyl group, anthracenyl group and the like; and the like can be mentioned.

The alkenyl group represented by R¹, R², R³, R⁴, R⁵ or R⁶ is linear or branched and preferably has 2 to 12, more preferably 2 to 6, carbon atoms. For example, allyl group and the like can be mentioned.

The aralkyl group represented by R¹, R², R³, R⁴, R⁵, R⁶, R⁷ or R⁸ preferably has 7 to 18, more preferably 7 to 12, carbon atoms. For example, benzyl group and the like can be mentioned.

The saturated hydrocarbon group represented by R⁹ is linear, branched or cyclic, and the carbon number thereof is preferably 1 to 12, more preferably 1 to 6, and, for example, an alkyl group such as methyl group, ethyl group, propyl group, isopropyl group, butyl group, isobutyl group, tert-butyl group, hexyl group, octyl group, dodecyl group and the like; a cycloalkyl group such as cyclopentyl group, cyclohexyl group and the like; and the like can be mentioned.

The aryl group represented by R⁹ preferably has 6 to 14, more preferably 6 to 10, carbon atoms and, for example, phenyl group, naphthyl group, anthracenyl group and the like can be mentioned.

The aralkyl group represented by R⁹ preferably has 7 to 18, more preferably 7 to 12, carbon atoms. For example, benzyl group and the like can be mentioned. These aralkyl groups may have substituent(s) and as such substituent, for example, a halogen atom such as chlorine atom and the like; an alkoxyl group having 1 to 6 carbon atoms such as methoxy group and the like; and the like can be mentioned.

The hydrocarbonoxy group represented by R⁹ is linear, branched or cyclic, and the carbon number thereof is preferably 1 to 13, and, for example, an alkoxyl group having 1 to 12 carbon atoms such as methoxy group, ethoxy group, propoxy group, isopropoxy group, butoxy group, isobutoxy group, tert-butoxy group, hexyloxy group, octyloxy group, dodecyloxy group and the like; a cycloalkyloxy group having 3 to 6 carbon atoms such as cyclopentyloxy group, cyclohexyloxy group and the like; an alkenyloxy group having 3 to 6 carbon atoms such as allyloxy group and the like; an aralkyloxy group having 7 to 13 carbon atoms such as benzyloxy group and the like; and the like can be mentioned.

The halogen atom represented by X is chlorine atom, fluorine atom, bromine atom or iodine atom, with particular preference given to chlorine atom.

The present invention is described in detail in the following.

The alkoxy-cyclic ether (I) can be produced according to an ordinary method. For example, alkoxy-cyclic ether (I) wherein R¹ and R² are hydrogen atoms can be easily obtained by reacting the corresponding 2,3-dihydrofuran with orthoformate in the presence of a Lewis acid according to the method described in JP-A-8-133997.

As specific examples of acid halide (II), for example acyl halides such as acetyl chloride, propionyl chloride, butyryl chloride, benzoyl chloride and the like; halogenated carbonates such as methyl chlorocarbonate, ethyl chlorocarbonate, propyl chlorocarbonate, isopropyl chlorocarbonate, butyl chlorocarbonate, isobutyl chlorocarbonate and the like; and the like can be mentioned. Of these, acetyl chloride, propionyl chloride, benzoyl chloride, methyl chlorocarbonate and ethyl chlorocarbonate are preferable.

The amount of acid halide (II) to be used in the present invention is preferably within the range of 0.8- to 5-fold mol, more preferably within the range of 1- to 3-fold mol, based on the alkoxy-cyclic ether (I), which is a starting material. The time for addition of acid halide (II) is generally within the range of 0.5 to 24 hr, and from the aspect of production efficiency, more preferably within the range of 1 to 10 hr.

This reaction is preferably performed in the presence of a solvent. Usable solvents are not particularly limited as long as they do not adversely affect the reaction and, for example, aromatic hydrocarbons such as benzene, toluene, xylene, chlorobenzene and the like; aliphatic hydrocarbons such as pentane, hexane, cyclohexane, octane and the like; halogenated hydrocarbons such as dichloromethane, chloroform, dichloroethane and the like; esters such as methyl acetate, ethyl acetate, n-propyl acetate, n-butyl acetate and the like; and the like can be mentioned. While the amount of the solvent to be used is not particularly limited, it is preferably within the range of 0.5- to 50-fold mass, and from the economical aspect, more preferably 1- to 10-fold mass, based on the alkoxy-cyclic ether (I).

The temperature of the reaction between alkoxy-cyclic ether (I) and acid halide (II) is preferably within the range of 0°C to 150°C, more preferably within the range of 40°C to 120°C. While the reaction time may vary depending on the reaction temperature, it is generally within the range of 1 to 24 hr after addition of acid halide (II).

In this reaction, halogenated unsaturated carbonyl compound (III) can be obtained by mixing alkoxy-cyclic ether (I), acid halide (II) and a solvent. To be specific, halogenated unsaturated carbonyl compound (III) can be obtained by adding acid halide (II), preferably adding dropwise to a mixture of alkoxy-cyclic ether (I) and the solvent. A catalyst may be added depending on the kind of the acid halide (II) to be used. As the catalyst usable here, organic bases such as pyridine and the like, and alcohols such as ethanol and the like can be mentioned. When the catalyst is to be added, its amount is preferably within the range of 0.1 to 20 mol%, more preferably within the range of 1 to 5 mol%, based on the alkoxy-cyclic ether (I).

After completion of the reaction, halogenated unsaturated carbonyl compound (III) can be isolated and purified by an ordinary isolation and purification operation, such as distillation, column chromatography and the like.

Halogenated unsaturated carbonyl compound (III), particularly the compound wherein n is 1, is a useful compound that can be led to cyclopropane monoacetal, which is a raw material for synthetic antibacterial agents. For example, the present inventors have clarified that halogenated unsaturated carbonyl compound (III) wherein n is 1, X is chlorine atom, R¹, R², R³, R⁴, R⁵ and R⁶ are hydrogen atoms, and R⁸ is ethyl group (4-chloro-2-ethoxymethylidenebutanal) can be led to 1-(diethoxymethyl)cyclopropanecarbaldehyde by reacting the compound with alcoholate, such as alkali metal ethoxide and the like. Note that 1-(diethoxymethyl)cyclopropanecarbaldehyde is used as a raw material for amino-substituted azaspiroalkane in WO02/14278, which is a raw material for synthetic antibacterial agents.

When halogenated unsaturated carbonyl compound (III) is used as a production raw material for cyclopropane monoacetal, which is a raw material for synthetic antibacterial agents, it is also possible to directly use the reaction solution (containing halogenated unsaturated carbonyl compound (II)) obtained in the present invention without taking out halogenated unsaturated carbonyl compound (II).

### Examples

The present invention is explained in detail in the following by referring to Reference Example and Examples, which are not to be construed as limitative.

### Reference Example 1

### Production of 3-(diethoxymethyl)-2-ethoxytetrahydrofuran

Triethyl orthoformate (1465 g, 9.89 mol) was added to a 3 L three-necked flask equipped with a thermometer and a stirrer, and cooled to 10°C to 12°C. Iron chloride (1.172 g, 0.00723 mol) was added as a catalyst and the mixture was stirred at the same temperature for 30 min. Then, 2,3-dihydrofuran (630 g, 8.99 mol) was added dropwise over 5.5 hr while maintaining the inner temperature at 10°C to 15°C, and the mixture was stirred at the same temperature for 1 hr. The reaction solution was analyzed by gas chromatography. As a result, 3-(diethoxymethyl)-2-ethoxytetrahydrofuran (1837 g, 8.42 mol) was produced. The yield based on 2,3-dihydrofuran was 93.7%. The reaction solution was transferred to a flask equipped with a distillation column (inner diameter 2.5 cm, height 30 cm) packed with ceramic Raschig ring, and distilled under reduced pressure to give 3-(diethoxymethyl)-2-ethoxytetrahydrofuran (1348.7 g, purity 99.7%) as a distilled fraction (a top temperature of 93°C to 94°C at a reduced pressure level of 0.67 kPa (5 mmHg)).

### Example 1

### Production of 4-chloro-2-ethoxymethylidenebutanal with acetyl chloride

3-(Diethoxymethyl)-2-ethoxytetrahydrofuran (20.01 g, 91.7 mmol) obtained by the method of Reference Example 1, toluene (46.02 g) and ethanol (126.2 mg, 2.74 mmol) were added to a 100 ml three-necked flask equipped with a thermometer, a stirrer and a dimroth condenser, and the mixture was heated to 90°C under a nitrogen atmosphere. Acetyl chloride (15.11 g, 192.5 mmol) was added dropwise to the mixture over 1 hr. After completion of the dropwise addition, the mixture was reacted at 80°C for 6 hr and analyzed by gas chromatography. As a result, 4-chloro-2-ethoxymethylidenebutanal (13.8 g, 84.9 mmol, yield 92.6%) was produced.
¹H-NMR(CDCl₃, ppm, TMS) δ:1.40 (t, 3H, J=7Hz), 2.65-2.80 (m, 2H), 3.50-3.65 (m, 2H), 4.20 (q, 2H, J=7Hz), 7.10 (s, 1H), 9.20 (s, 1H).

### Example 2

### Production of 4-chloro-2-ethoxymethylidenebutanal with acetyl chloride

The operation was conducted in the same manner as in Example 1 except that the acetyl chloride was added over 4 hr. As a result, the yield of 4-chloro-2-ethoxymethylidenebutanal was 89.7%.

### Example 3

### Production of 4-chloro-2-ethoxymethylidenebutanal with ethyl chlorocarbonate

3-(Diethoxymethyl)-2-ethoxytetrahydrofuran (20.03 g, 91.8 mmol) obtained by the method of Reference Example 1, toluene (46.0 g) and pyridine (0.22 g, 2.8 mmol) were added to a 100 ml three-necked flask equipped with a thermometer, a stirrer and a dimroth condenser, and the mixture was heated to 100°C to 106°C under a nitrogen atmosphere. Ethyl chlorocarbonate (19.92 g, 183.5 mmol, 2-fold mol relative to 3-(diethoxymethyl)-2-ethoxytetrahydrofuran) was added dropwise to the mixture over 1 hr. After completion of the dropwise addition, the mixture was maintained at the same temperature for 6 hr, and the reaction solution was analyzed by gas chromatography. As a result, the conversion ratio of 3-(diethoxymethyl)-2-ethoxytetrahydrofuran was 100%, and 4-chloro-2-ethoxymethylidenebutanal (14.3 g, 87.9 mmol, yield 95.8%) was produced.

### Example 4

### Production of 4-chloro-2-ethoxymethylidenebutanal with ethyl chlorocarbonate

The operation was conducted in the same manner as in Example 3 except that the amount of ethyl chlorocarbonate was 1.3-fold mol relative to the starting material. As a result, the conversion ratio of 3-(diethoxymethyl)-2-ethoxytetrahydrofuran was 97.1%, and 4-chloro-2-ethoxymethylidenebutanal was obtained in a yield of 90.5%.

### Industrial Applicability

The halogenated unsaturated carbonyl compound that can be produced by the method of the present invention is useful as a raw material for cyclopropane monoacetal which is a useful compound as a raw material for synthetic antibacterial agents.

This application is based on a patent application No. 2004-104866 filed in Japan, the contents of which are incorporated in full herein by this reference.

## Claims

1. A method of producing a halogenated unsaturated carbonyl compound represented by the formula (III) 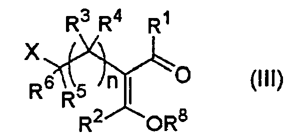 wherein R¹, R², R³, R⁴, R⁵ and R⁶ each independently represents a hydrogen atom, a saturated hydrocarbon group optionally having substituent(s), an aryl group optionally having substituent(s), an alkenyl group or an aralkyl group, R⁸ represents a saturated hydrocarbon group optionally having substituent(s), an aryl group optionally having substituent(s) or an aralkyl group, X represents a halogen atom, and n represents 1 or 2,
which comprises reacting an alkoxy-cyclic ether represented by the formula (I) 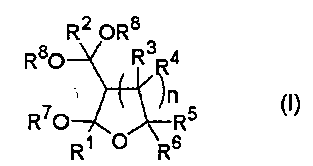 wherein R¹, R², R³, R⁴, R⁵, R⁶, R⁸ and n represent as defined above, and R⁷ represents a saturated hydrocarbon group optionally having substituent(s), an aryl group optionally having substituent(s) or an aralkyl group, with an acid halide represented by the formula (II) 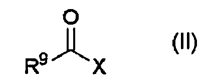 wherein R⁹ represents a saturated hydrocarbon group, an aryl group, an aralkyl group optionally having substituent(s) or a hydrocarbonoxy group, and X represents as defined above.
